# EUROPÄISCHE PATENTSCHRIFT

(11) **EP 0 578 912 B1**
(45) Veröffentlichungstag und Bekanntmachung des Hinweises auf die Patenterteilung: **15.09.1999**
(21) Anmeldenummer: 93102511.8
(22) Anmeldetag: 18.02.1993
(51) Int. Cl.: C12N 1/20, C12N 1/28, A23K 3/03

(54) **Verwendung eines wässrigen Nährmediums zur Vermehrung von homofermentativen Milchsäurebakterien unter unsterilen Bedingungen zum Einsatz als Siliermittel bei der Gärfutterbereitung**
Use of a liquid culture medium for the multiplication of homofermentative lactic acid bacteria under non-sterile conditions to apply in silage feed preparation
Usage d'un milieu de culture nutritif liquide pour la multiplication de bactéries lactiques homofermentatives dans des conditions non-stériles à appliquer dans la préparation de fourrage ensilé

(30) Priorität: 27.02.1992 DE 4206057
(43) Veröffentlichungstag der Anmeldung: 19.01.1994
(73) Patentinhaber: SANOFI-CEVA GmbH, 40472 Düsseldorf (DE)
(72) Erfinder: Beck-Sirch, Therese, W-8000 München 40 (DE); Gross, Friedrich, Dr., 82205 Gilching (DE)
(74) Vertreter: Türk, Gille, Hrabal

(56) Entgegenhaltungen:
- EP-A- 0 071 858
- EP-A- 0 399 385
- BE-A- 478 719
- GB-A- 2 159 388
- DATABASE WPI Section Ch, Week 8451, Derwent Publications Ltd., London, GB; Class D16, AN 84-314746 & JP-A-59 196 087 (RYOSHOKU KENKYUKAI) 7. November 1984

## Beschreibung

Die Erfindung betrifft die Verwendung eines wäßrigen Nährmediums enthaltend Kohlenstoffquellen, wobei Monosaccharide in einer Menge von 2 bis 6 Gew.-%, bezogen auf die Wassermenge vorliegen, Stickstoffquellen, Macroelemente und Spurenelemente, Alkalisalze der Essigsäure in einer solchen Menge, daß von 0,7 bis 2,0 Gew.-% der CH₃COO-Gruppe bezogen auf die Wassermenge vorliegen und Pufferverbindungen in einer solchen Menge, daß bei zweitägigem Stehen bei 20°C unter Zusatz von Milchsäurebakterien der pH-Wert nicht kleiner als 4 wird, zur Vermehrung von homofermentativen Milchsäurebakterien unter unsterilen Bedingungen. Die Erfindung betrifft weiterhin die Verwendung der so erhaltenen Milchsäurebakterienkulturen als Silierhilfsmittel sowie ein Trockennährmedium-Milchsäurebakterienkombinationspräparat.

Milchsäure erzeugende Bakterien spielen beim Einsilieren von Grünfutter, welches eine außerordentliche wirtschaftliche Bedeutung hat, eine wichtige Rolle, da die Milchsäuregärung ein wesentlicher Vorgang beim Einsilieren von Grünfutter ist.

So beschreibt die EP-A-0 250 786 ein Verfahren zum Einsäuern von Grünfutter unter Verwendung von Bakterienpräparaten aus Milchsäure erzeugenden Bakterien. Dabei wird dem Anwender (Landwirt) ein Bakterienpräparat hoher Keimdichte zur Verfügung gestellt, welches in bekannter Weise mit Hand, Dosiergeräten, Pulverspritzen und dergleichen dem einzusilierenden Gut zugesetzt wird.

Die OE-A-262 040 beschreibt ein Verfahren zur Herstellung eines als Silierhilfsmittel bei der Futterbereitung geeigneten Milchsäurebakterienpräparates welches dem Anwender (Landwirt) als gefriergetrocknetes Trockenpräparat mit hoher Keimdichte von ca. 10¹² vermehrungsfähigen Keimen pro 1 g Präparat zur Verfügung gestellt wird.

Daneben sind Kombinationspräparate von Milchsäurebakterien mit Alkali- und/oder Erdalkaliformiaten (EP-A-0 399 385) bekannt.

Diesen Erfindungen ist gemeinsam, daß dem Landwirt ein Milchsäurebakterienpräparat mit hoher Ausgangskeimdichte zur Verfügung gestellt wird, um eine ausreichende Keimzahl im zu silierenden Gut von etwa 10⁵ bis 10⁶ Keimen pro 1 g Trockensubstanz zu erzielen. Die aufwendige Herstellung solcher Milchsäurebakterienpräparate mit hoher Keimdichte führt dazu, daß dem Endverbraucher (Landwirt) bislang nur relativ teure Präparate auf dem Markt zugänglich sind. Die Herstellung einer als Silierhilfsmittel geeigneten Milchsäurebakterienkultur mit hoher Keimdichte durch den Endverbraucher selbst scheiterte bislang an der Sterilitätsbedingung, die der Endverbraucher (Landwirt) aufgrund des damit verbundenen Aufwands nicht realisieren konnte.

Der vorliegenden Erfindung lag daher die Aufgabenstellung zugrunde, ein wäßriges Nährmedium für die Vermehrung von Milchsäurebakterien bereitzustellen, daß folgenden Bedingungen genügt:
a) Die Vermehrung der Milchsäurebakterien soll unter einfachsten unsterilen Bedingungen, wie sie auf jedem landwirtschaftlichen Betrieb gegeben sind, möglich sein.
b) Die Vermehrung der Milchsäurebakterien soll zu einer solchen Keimdichte führen, die den Einsatz als Starterkulturen bei der Gärfutterbereitung möglich macht.
c) Die gewachsenen Milchsäurebakterien sollen eine für die praktischen Verhältnisse erwünschte ungewöhnlich lange Lebensfähigkeit von mindestens einer Woche aufweisen.
d) Das dem Landwirt zur Verfügung gestellte Ausgangsmaterial soll ein Trockennährmedium-Milchsäurebakterienkombinationspräparat sein, das nach luftdichtem Abschluß unter Normalbedingungen eine möglichst hohe Lagerstabilität von über einem halben Jahr aufweist.
e) Die erhaltenen Milchsäurebakterienkulturen sollen mit handelsüblichen Verteilergeräten (Spritztüten) dosierbar und verteilbar sein.
f) Die vom Landwirt selbst gezüchteten Milchsäurebakterienkulturen sollen nach Ausbringung auf das Siliergut mindestens eben so schnell im Silo zur Vermehrung kommen als lyophilisierte Kulturen.
g) Das Verfahren soll für den Landwirt deutlich wirtschaftlicher sein als die bisherige Verwendung von Siliermitteln auf Milchsäurekulturenbasis gleicher Keimdichte.

D. h. es soll ein Nährmedium bereitgestellt werden, das es dem Landwirt ermöglicht, anstelle von sehr teuren, gefriergetrockneten Milchsäurebakterienkulturen mit hohem Keimgehalt, mit Hilfe eines kostengünstigen, im folgenden beschriebenen Nährmedium mit extrem niedriger Ausgangskeimdichte, selber auf einfache Weise unter unsterilen Bedingungen Milchsäurebakterienkulturen mit hoher Keimdichte herzustellen, welche als Silierhilfsmittel verwendet werden könnten.

Es wurde nun überraschend gefunden, daß diese Aufgabe durch die Verwendung eines wäßrigen Nährmediums enthaltend Kohlenstoffquellen, wobei Monosaccharide in einer Menge von 2 bis 6 Gew.-%, bezogen auf die Wassermenge vorliegen, Stickstoffquellen, Macroelemente und Spurenelemente, Alkalisalze der Essigsäure in einer solchen Menge, daß von 0,7 bis 2,0 Gew.-% der CH₃COO-Gruppe, bezogen auf die Wassermenge vorliegen, und Pufferverbindungen in einer solchen Menge, daß bei zweitägigem Stehen bei 20°C unter Zusatz von Milchsäurebakterien der pH-Wert nicht kleiner als 4 wird, zur Vermehrung von homofermentativen Milchsäurebakterien unter unsterilen Bedingungen gelöst werden kann.

Geeignete Kohlenstoffquellen sind neben Monosacchariden wie Glucose, Fructose und Galactose z. B. Saccharose, Melasse, Laktose, Malzextrakte, Dextrine und/oder Stärke. Bevorzugt sind leicht und direkt fermentierbare Kohlenhydrate, die nicht erst enzymatisch in Monomere gespalten werden müssen, also Monosaccharide. Besonders bevorzugt ist die Verwendung von Glucose. Als Stickstoffquellen eignen sich üblicherweise z. B. Proteine, Peptone, Sojamehl, Hefeextrakte und Ammoniumsulfat. Geeignete Macroelemente sind z. B. Phospate, Kalium oder Magnesium-Salze. Als Spurenelemente können üblicherweise z. B. Mangan, Molybdän, Zink, Kupfer oder Kobalt in Form ihrer Salze zugesetzt werden. Das Nährmedium enthält Alkalisalze der Essigsäure in einer solchen Menge, daß von 0,7 bis 2,0 Gew.-% der CH₃COO-Gruppe bezogen auf die Wassermenge vorliegen. Diese Mengenangabe ist so zu verstehen, daß wenn z. B. als Alkalisalz der Essigsäure das bevorzugt verwendete Natriumacetat in einer Menge von 1,0 Gew.-% dem Nährmedium zugesetzt wurde, 0,7 Gew.-% der CH₃COO-Gruppe im wäßrigen Nährmedium vorliegen. Die Bezeichnung CH₃COO-Gruppe wurde aus formalen Gründen der exakten Mengendefinition gewählt, da die Acetatanionen in wäßriger Lösung sowohl in unprotonierter als auch in protonierter Form als Essigsäure vorliegen können.

Als homofermentative Milchsäurebakterien werden bevorzugt homofermentative Vertreter des Genus Lactobacillus und/oder Pediococcus, besonders bevorzugt solche der Spezies Lactobacillus plantarum DSM 3676 und/oder Lactobacillus plantarum DSM 3677 verwendet. Dabei ist unter homofermentativ die Eigenschaft, aus Hexosen kein Gas bilden zu können, zu verstehen.

Nach der neuesten Nomenklatur (O. Kandler und N. Weiss: The Genus Lactobacillus. - BERGEY's - Manual of Systematic Bacteriology, (1986) Vol. 2, The Williams and Wilkings Co., Baltimore) werden die ursprünglich als homofermentativ bezeichneten Milchsäurebakterien, die zu den homofermentativen Vertretern des Genus Lactobacillus und/oder des Genus Pediococcus gehören, nunmehr als fakultativ heterofermentative Lactobacillen angesprochen.

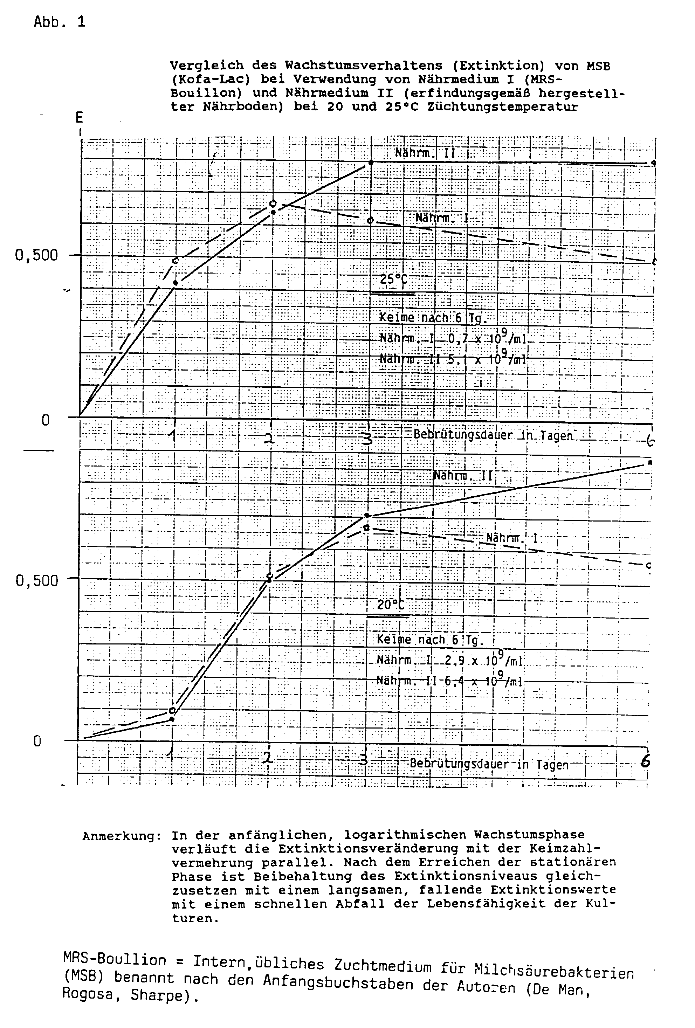

Die überraschende Möglichkeit der Vermehrung der Milchsäurebakterien unter nicht sterilen Bedingungen wird durch die Kombination der Merkmale
- sehr hohe Monosaccharidkonzentration
- selektiv wirksame Acetatkonzentrationen zur Hemmung von Nicht-Lactobacillen (sowie Streptokokken)
- unüblich hohen Gehalt an puffernd wirkenden Verbindungen ermöglicht. Aus entsprechenden Nährbodeneignungsversuchen (s. Abb. 1) ergab sich überraschender Weise, daß erst die Kombination dieser Züchtungsbedingungen zum gewünschten Erfolg, nämlich der Erzielung hoher Keimdichten mit langer Lebensfähigkeit sowie Unterdrückung von Fremdinfektionen bei unsterilen Kulturbedingungen führt. Die marktgängigen Nährböden für Milchsäurebakterienkulturen sind für die angegebenen Zwecke ungeeignet, da sie
- unter unsterilen Bedingungen das Wachstum von Fremdkeimen nicht unterdrücken,
- die Lebensfähigkeit der gewachsenen Kulturen nicht über längeren Zeitraum gewährleisten.

Die Versuche der Erfinder zeigen, daß die Erhöhung der Monosaccharidkonzentration einen langen zeitlichen Betriebs- und Baustoffwechsel der wachsenden Bakterien ermöglicht. Der unüblich hohe Gehalt an puffernd wirkenden Verbindungen führt zu einer starken Pufferung des wäßrigen Kulturmediums. Die hohen Acetatkonzentration unterdrücken die Entwicklung von Fremdkeimen.

Die untere Grenze des Monosaccharidgehalts des wäßrigen Nährmediums beträgt, bezogen auf die Wassermenge, mindestens etwa 2 Gew.-% Monosaccharid, bevorzugt mindestens etwa 3 Gew.-%. Die obere Grenze des Monosaccharidgehalts beträgt höchstens etwa 6 Gew.-%, bevorzugt höchstens etwa 5 Gew.-%. Besonders bevorzugt liegt der Monosaccharidgehalt bei etwa 4 Gew.-%. Besonders bevorzugt ist dabei die Verwendung von Glucose als Monosaccharid.

Die untere Grenze des CH₃COO-Gruppengehalts des wäßrigen Nährmediums beträgt, bezogen auf die Wassermenge, mindestens etwa 0,7 Gew.-%, bevorzugt mindestens etwa 1,0 Gew.-%. Die obere Grenze des CH₃COO-Gruppengehalts des wäßrigen Nährmediums beträgt, bezogen auf die Wassermenge, höchstens etwa 2,0 Gew.-%, bevorzugt höchstens etwa 1,6 Gew.-%. Besonders bevorzugt beträgt der CH₃COO-Gruppengehalt des wäßrigen Nährmediums, bezogen auf die Wassermenge, etwa 1,4 Gew.-%. Besonders bevorzugt ist dabei die Verwendung von Natriumacetat zum Erreichen der gewünschten CH₃COO-Gruppenkonzentrationen.

Die starke Pufferung der bei der Vermehrung der Milchsäurebakterien gebildeten Milchsäure wird durch einen unüblich hohen Zusatz von Pufferverbindungen, erreicht. Die Pufferverbindungen werden in dem wäßrigen Nährmedium in einer solchen Menge zugesetzt, daß bei zweitägigem Stehen bei 20 °C unter Zusatz von Milchsäurebakterien der pH-Wert nicht kleiner als 4 wird.

In dieser Definition kann der Zusatz von Milchsäurebakterien an sich in beliebiger Weise erfolgen, entscheidend ist, daß der pH-Wert während der Züchtung nicht unter 4 sinkt.

Bevorzugte Pufferverbindungen sind Hydrogenphosphatsalze. Diese können allein oder in Kombination mit anderen Pufferverbindungen zugesetzt werden. Besonders bevorzugt wird Dikaliumhydrogenphosphat zugesetzt. Dabei beträgt die untere Grenze des Dikaliumhydrogenphosphatgehalts des wäßrigen Nährmediums zweckmäßig, bezogen auf die Wassermenge mindestens etwa 0,4 Gew.-%, bevorzugt mindestens etwa 0,6 Gew.-%. Die obere Grenze des Dikaliumhydrogenphosphatgehalts des wäßrigen Nährmediums beträgt zweckmäßig, bezogen auf die Wassermenge, höchstens etwa 1,2 Gew.-%, bevorzugt höchstens etwa 1,0 Gew.-%. Besonders bevorzugt beträgt die Dikaliumhydrogenphosphatmenge etwa 0,8 Gew.-%. Auch das Acetat-Ion im Natriumacetat übernimmt eine Pufferfunktion, da die Milchsäure eine stärkere Säure als die Essigsäure ist.

Die untere Grenze der Keimdichte der Milchsäurebakterien im wäßrigen Nährmedium gemäß der Erfindung beträgt bei Beginn der Züchtung zweckmäßig mindestens etwa 1 X 10⁴/g Wasser, bevorzugt mindestens etwa 1 X 10⁵/g Wasser. Die obere Grenze der Ausgangskeimdichte beträgt zweckmäßig höchstens etwa 1 X 10⁷/g Wasser, bevorzugt höchstens etwa 1 X 10⁶/g Wasser. Besonders bevorzugt beträgt die Ausgangskeimdichte der Milchsäurebakterien etwa 5 X 10⁵/g Wasser.

Die Erfindung betrifft weiterhin ein Trockennährmedium-Milchsäurebakterienkombinationspräparat, dadurch gekennzeichnet, daß es Alkalisalze der Essigsäure, Glucose, Pufferverbindungen, homofermentative Milchsäurebakterien in einer Menge von etwa 2,5 x 10⁴ bis 2,5 x 10⁷ pro 100 g Glucose sowie weitere Kohlenstoffquellen, Stickstoffquellen, Macroelemente und Spurenelemente in solchen Mengen enthält, daß nach Lösen des Präparats in Wasser das wäßrige Nährmedium nach Anspruch 1 erhalten wird. Bevorzugt ist CH₃-COO- als Natriumacetat enthalten.

Die untere Grenze der Keimdichte der Milchsäurebakterien beträgt mindestens etwa 2,5 X 10⁴, bevorzugt mindestens etwa 2,5 X 10⁵ Keime pro 100 g Glucose. Die obere Grenze der Keimdichte der Milchsäurebakterien beträgt höchstens etwa 2,5 X 10⁷ Keime pro 100 g Glucose, bevorzugt höchstens etwa 2,5 X 10⁶ Keime pro 100 g Glucose. Besonders bevorzugt beträgt die Keimdichte der Milchsäurebakterien etwa 1,25 X 10⁶ Keime pro 100 g Glucose.

Zum Aufschliessen von Stärke und Rohfasern im Silofutter können dem Trockennährmedium-Milchsäurebakterienkombinationspräparat gemäß der Erfindung noch verschiedene Enzyme wie Amylase, Glukosidase, Amylo-Glukosidase, Zellulase und ähnliches zugesetzt werden.

Das Trockennährmedium-Milchsäurebakterienkombinationspräparat gemäß der Erfindung wird dem Endverbraucher zweckmäßig in Form einer Mischung von steril verpacktem Trockennährmedium und Milchsäurebakterienpräparat bereitgestellt. Das Verkaufspräparat liegt üblicherweise als Pulver vor, welches eine sehr gute Lagerstabilität von mindestens einem halben Jahr hat, wie die folgenden Versuche zeigten:

Prüfung der Lagerstabilität des erfindungsgemäßen sterilen Trockennährmedium-Milchsäurebakterienkombinationspräparates.

### Methodische Voraussetzungen:

Zur Prüfung der Lagerstabilität des Trockennährmedium-Bakterienkombinationspräparates war die Aufteilung des Präparates unmittelbar nach Herstellung in eine größere Zahl von aliquoten Parallelproben erforderlich, die nach luftdichtem Abschluß im Dunkeln bei Zimmertemperatur (20 °C) und im Kühlschrank (4 bis 5 °C) verschieden lang aufbewahrt wurden. Um zu entscheiden, ob eventuell ein Keimdichteabfall die Wachstumsgeschwindigkeit beeinflußt, erfolgte eine Bestimmung des Keimgehaltes sowie Versuche zur Ermittlung der Wachstumsgeschwindigkeit (Extinktionsmessung bei verschiedener Bebrütungsdauer) unter exakt gleichen Bedingungen (gewählte Temperatur 25 °C) der verschieden lang gelagerten Parallelproben. Das Ergebnis dieses Lagerungsversuches zeigt Tab. 1 (E = Extinktion, log der Lichtdurchlässigkeit der 1 : 20 mit destilliertem Wasser verdünnten Kulturflüssigkeit bei 578 nm; E = Maß für die Wachstumsintensität).

**Tab. 1**

| Prüfung der Lagerstabilität des Nährboden-Bakterienpräparates | | | | | | | | |
|---|---|---|---|---|---|---|---|---|
| Lagerdauer in Tagen | 0 | | 21 | | 56 | | 112 | |
| Aufbewahrung b. | 20°C | 4°C | 20°C | 4°C | 20°C | 4°C | 20°C | 4°C |
| Keimgehalt 10⁶/ml | 0,8 | | 0,61 | 0,72 | 0,41 | 0,50 | 0,14 | 0,41 |
| E b. Kultur von: | | | | | | | | |
| 24 h | 0,445 | | 0,295 | 0,335 | 0,300 | 0,310 | 0,120 | 0,245 |
| 48 h | 0,980 | | 0,970 | 0,990 | 0,980 | 0,990 | 0,890 | 0,995 |
| 72 h | 1,090 | | 1,080 | 1,080 | 1,070 | 1,070 | 1,090 | 1,100 |

Aus den Tabellenwerten ergibt sich, daß auch bei längerer Aufbewahrung des Trockennährmedium-Bakterienkombinationspräparat gemäß der Erfindung der Ausgangskeimgehalt bei Zimmertemperatur nur vergleichsweise gering, bei Kühlschranklagerung nur vergleichsweise sehr gering zurückgeht. Dieser Keimabfall bewirkt nur bei einer Bebrütungsdauer bis zu 24 Stunden eine merkliche Verzögerung der Wachstumsgeschwindigkeit der bei 20 °C gelagerten und einen unerheblichen Abfall der bei 4 °C gelagerten Proben nach längerer Lagerungsdauer gegenüber der Kontrolle zum Zeitpunkt Null. Nach zwei Tagen Bebrütungsdauer sind diese Unterschiede in der Wachstumsstärke nahezu, nach drei Tagen vollständig, ausgeglichen.

Das wäßrige Nährmedium zur Vermehrung von Milchsäurebakterien gemäß der Erfindung wird zweckmäßig durch Versetzen des Trockennährmedium-Milchsäurebakterienkombinationspräparats gemäß der Erfindung mit Leitungswasser hergestellt. Die Vermehrung der Milchsäurebakterien erfolgt dann durch Bebrütung bei Raumtemperatur. Unter Raumtemperatur im Sinne der Erfindung wird ein Temperaturbereich von etwa 17 °C bis 24°C, bevorzugt von 19 °C bis 22 °C verstanden Ein Vergleich des Wachstumgsverhaltens (Extinktion) von Milchsäurebakterien (Handelspräparat Kofa-Lac®) bei Verwendung von Nährmedium I (MRS-Boullion) und dem wäßrigen Nährmedium gemäß der Erfindung bei 20 und 25° Bebrütungstemperatur ist in Abb. 1 gezeigt. Bei 25 °C erreicht das Nährmedium I seine maximale Keimdichte nach zwei Tagen, die dann rasch abfällt. Demgegenüber erreicht das Nährmedium II gemäß der Erfindung eine höhere Keimdichte nach drei Tagen, die dann nur langsam abfällt. Nach sechs Tagen Bebrütungsdauer beträgt die Keimdichte des Nährmediums I 0,7 X 10⁹/ml, während die Keimdichte des erfindungsgemäßen Nährmediums II 5,1 X 10⁹/ml beträgt.

Bei 20 °C Bebrütungstemperatur erreicht das Nährmedium I seine maximale Keimdichte etwa am dritten Tag der Bebrütung. Danach folgt ein schneller Abfall der Lebensfähigkeit der Kulturen. Demgegenüber nimmt die Keimdichte bei dem Nährmedium II gemäß der Erfindung auch nach dem dritten Tag noch zu. Nach dem sechsten Tag der Bebrütung beträgt die Keimdichte des Nährmediums I 2,9 X 10⁹/ml, während die Keimdichte des Nährmediums II gemäß der Erfindung 6,4 X 10⁹/ml beträgt.

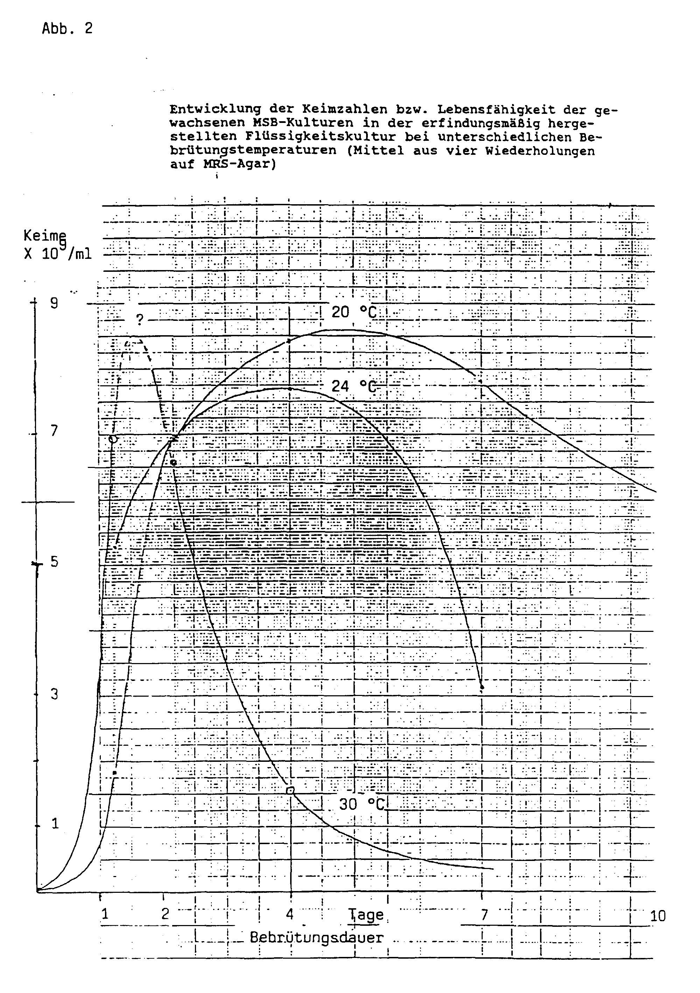

Versuche der Erfinder zeigen, daß auch bei höheren Temperaturen als Raumtemperatur bebrütet werden kann (Abb. 2). Dadurch wird die Keimvermehrung anfänglich sehr stark beschleunigt, die Keimdichte fällt jedoch bei längerer Bebrütung auch wieder sehr schnell ab.

Eine einfache Bebrütung der angesetzten Kulturen bei konstanten Temperaturen im Bereich zwischen 22 bis 35 °C über einen längeren Zeitraum ist z. B. durch Einsatz eines Heizstabes für Aquarien möglich. Durch die erfindungsgemäße Verwendung des wäßrigen Nährmediums wird eine Kulturflüssigkeit mit hoher Keimdichte von ca. 5 bis 8 x 10⁹ Zellen/ml an Lactobacillen erzeugt, die darüber hinaus über einen relativ langen Zeitraum von bis zu 10 Tagen brauchbar bleibt, was zu Vorteilen bei der Anwendung führt. Kann die Kulturflüssigkeit innerhalb von 2 bis 10 Tagen nicht verwendet werden (z. B. wegen ungünstiger Witterungsbedingungen für das Einsilieren), so besteht die Möglichkeit das Gefäß mit Kulturflüssigkeit (im Normalfall 1 bis 10 l) bei Kühlschrankaufbewahrung eine weitere Woche ohne merklichen Keimabfall (< 10 %) zu konservieren. Die so hergestellten Milchsäurebakterienkulturen sind in ihrer Wirksamkeit zumindestens gleich oder besser zum Einsilieren geeignet als die handelsüblichen getrockneten (lyophilisierten) Milchsäurebakterienpräparate gleicher Keimdichte, weil die gewachsenen Milchsäurebakterienkulturen im Gegensatz zu Trockenprodukten keine Lac-Phase benötigen.

Tabelle 2 vergleicht das Wachstumsverhalten von Milchsäurebakterien (Kofa-Lac®, handelsübliches gefriergetrocknetes Milchsäurebakterienpräparat) bei Verwendung von Impfmaterial im gefriergetrocknetem Zustand und nach Wachstum in der erfindungsgemäß hergestellten Kulturflüssigkeit in handelsüblicher MSB-Boullion (MRS) bei gleicher Impfmenge (1,5 X 10⁵/ml) und 28 °C Bebrütungstemperatur:

**Tab. 2**

| Kultur | 15 Std. | | 21,5 Std. | | | 31 Std. | | |
|---|---|---|---|---|---|---|---|---|
| | pH | E¹⁾ | pH | E¹⁾ | Keime 10⁹/ml | pH | E¹⁾ | Keime 10⁹/ml |
| gefr.getr.²⁾ | 7,09 | <0,010 | 5,44 | 0,170 | 0,8 | 3,81 | 0,715 | 4,1 |
| Flü.-Kultur³⁾ | 6,54 | 0,054 | 4,37 | 0,437 | 3,2 | 3,79 | 0,735 | 4,2 |

| | | | | | | | | |
|---|---|---|---|---|---|---|---|---|
| Bemerkung: 1) E = Extinktion der 1 : 20 mit destilliertem Wasser verdünnten Kulturflüssigkeit bei 578 nm als Maß für die Wachstumsstärke (Trübung) | | | | | | | | |
| 2) handelsübliches lyophilisiertes Produkt | | | | | | | | |
| 3) 43-stündige Kultur der erfindungsgemäß hergestellten Flüssigkeitskultur | | | | | | | | |

Die anfängliche Wachstums- und Säuerungsgeschwindigkeit ist bei gleicher Ausgangskeimdichte und bis zu 31 Stunden Kulturdauer bei Impfung mit gewachsenen Kulturen deutlich höher bzw. schneller als bei Verwendung von gefriergetrockneten Kulturen

Der optimale Einsatzbereich von Milchsäurebakterienkulturen als Siliermittel ist bekannt und vorstehend beschrieben. Er ist abhängig vom Gehalt des Siliergutes an vergärbaren Kohlenhydraten, der Pufferkapazität und dem epiphytischen Keimbesatz. Die Wirkung der Milchsäurebakterienzusätze kann durch die Kombination mit geeigneten Konservierungsstoffen, (z. B. Natriumformiat) soweit verbessert werden, daß auch bei schwer silierbarem Material ein Erfolg gewährleistet ist. Mit erfindungsgemäß angezogenen Milchsäurebakterienkulturen wurden nach einer Bebrütungsdauer von zwei Tagen vergleichende Silierversuche mit Kofa-Lac (eingetragenes Warenzeichen; Handelspräparat gemäß dem Stand der Technik) und bei zwei unterschiedlichen Impfstärken durchgeführt. Die Ergebnisse dieser Weckglassilierung der Futterarten A) bis D) sind in Tabelle 3 zusammengestellt.

Da alle Futterarten sich als vergleichsweise gut silierbar, zumindestens in der Anfangsgärung erwiesen (Anlage während einer langanhaltenden Schönwetterperiode) und einen ähnlichen Gärverlauf zeigten, schien eine Mittelbildung aller 4 Versuche gerechtfertigt.

Im Mittel der vergleichenden Silierversuche mit vier Futterarten sind die anfängliche Säuerungsgeschwindigkeit sowie die Gärverluste als Maß für den Siliererfolg bei Einsatz von Flüssigkeitskulturen gesichert gegenüber Einsatz gefriergetrockneter MSB-Kulturen gleicher Keimdichte verbessert. Im Vergleich zur Kontrolle führt der Zusatz von MSB-Kulturen in Abhängigkeit von der Impfstärke bei allen vier Futterarten zu einer deutlichen Verringerung der Gärverluste.

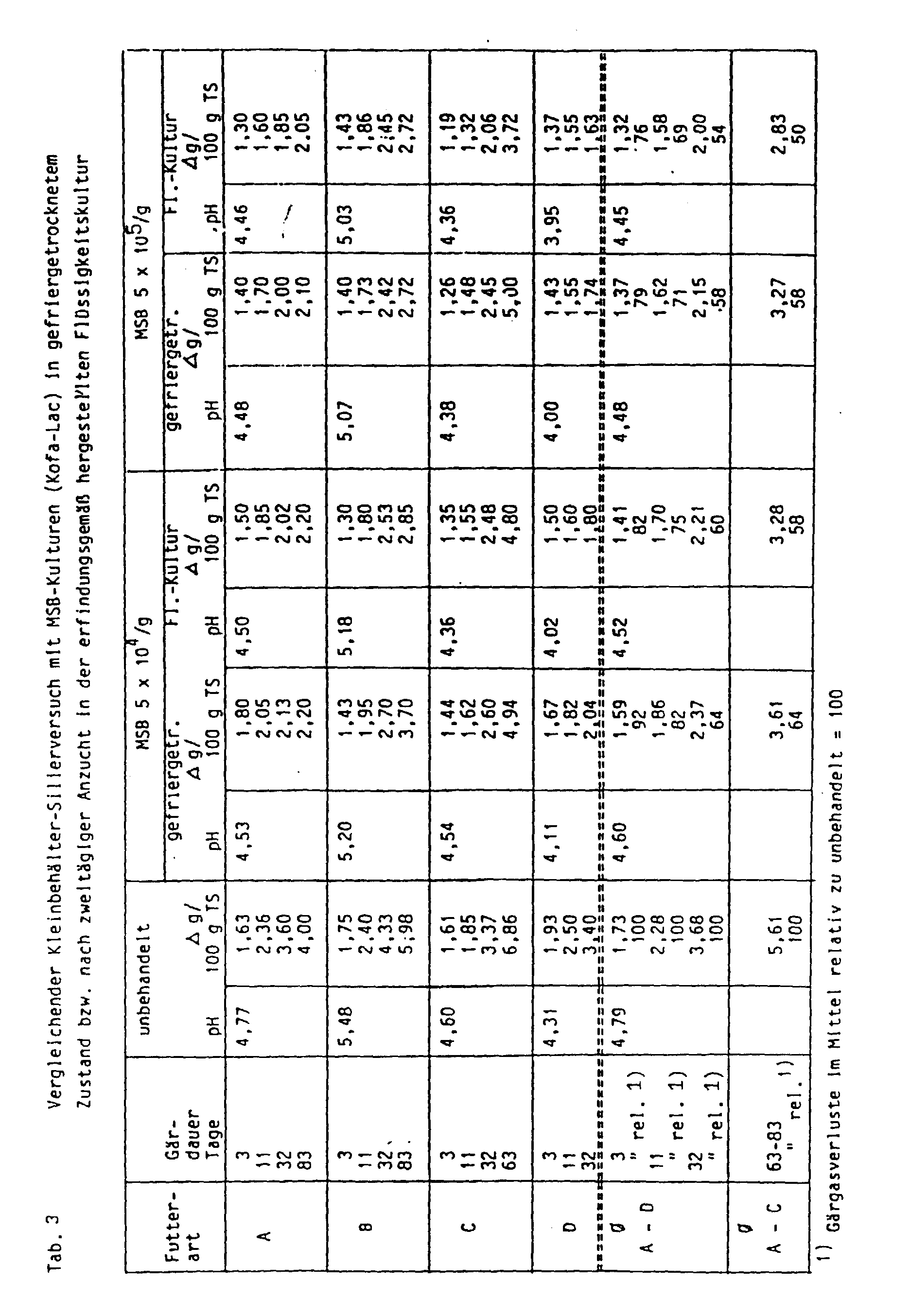

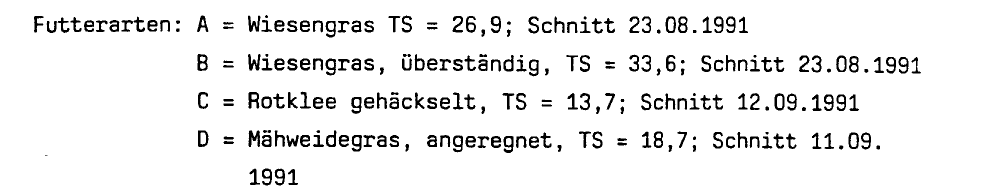

- Bedingungen:: 500 g Frischsubstanz in 1 1 Kleinbehälter, Gärtemperatur 25 °C 4 bzw. 3 Wiederholungen nach Öffnung einer Kontrolle nach 3 Tagen für pH-Bestimmung. Messung der Gärverluste (Δg/100 g TS) durch Differenzwägung. Impfmenge der gefriergetrockneten MSB und der MSB aus Flüssigkeitskultur mit gleicher Keimdichte.

### Anwendungsbeispiel:

Ein Trockennährmedium wird durch das Zusammengeben folgender Komponenten in den gegebenen Mengen hergestellt:
a) Universalpepton 10 g
b) Glucose 40 g
c) Fleischextrakt (Trockenpulver) 5 g
d) Hefeextrakt 5 g
e) Diammoniumhydrogencitrat 2,5 g
f) Na-Acetat (wasserfrei) 19 g
g) Dikaliumhydrogenphosphat 8 g
h) Magnesiumsulfat 0,15 g
i) Mangansulfat 0,05 g

Durch Zugabe von 50 mg Kofa-Lac (handelsübliches gefriergetrocknetes Milchsäurebakterienpräparat) erhält man ein Trockennährmedium-Milchsäurebakterienkombinationspräparat. Das Zusammengeben von Trockennährmedium und Milchsäurebakterienpräparat erfolgt zweckmäßig durch den Hersteller, kann aber auch durch den Anwender erfolgen.

Das so erhaltene Trockennährmedium-Milchsäurebakterienkombinationspräparat wird zweckmäßig mit einem Liter Leitungswasser versetzt und bei Raumtemperatur bebrütet.

## Patentansprüche

1. Verwendung eines wäßrigen Nährmediums enthaltend Kohlenstoffquellen, wobei Monosaccharide in einer Menge von 2 bis 6 Gew.-%, bezogen auf die Wassermenge vorliegen, Stickstoffquellen, Macroelemente und Spurenelemente, Alkalisalze der Essigsäure in einer solchen Menge, daß von 0,7 bis 2,0 Gew.-%, der CH₃COO-Gruppe, bezogen auf die Wassermenge vorliegen und Pufferverbindungen in einer solchen Menge, daß bei zweitägigem Stehen bei 20°C unter Zusatz von Milchsäurebakterien der pH-Wert nicht kleiner als 4 wird, zur Vermehrung von homofermentativen Milchsäurebakterien unter unsterilen Bedingungen.

2. Verwendung nach Anspruch 1, dadurch gekennzeichnet, daß als Monosaccharid Glucose verwendet wird.

3. Verwendung eines wäßrigen Nährmediums nach einem oder mehreren der Ansprüche 1 bis 2, dadurch gekennzeichnet, daß die homofermentativen Milchsäurebakterien zu homofermentativen Vertretern des Genus Lactobacillus und/oder Pediococcus gehören.

4. Verwendung eines wäßrigen Nährmediums nach einem oder mehreren der Ansprüche 1 bis 3, dadurch gekennzeichnet, daß die homofermentativen Milchsäurebakterien Lactobacillus plantarum DSM 3676 und/oder Lactobacillus plantarum DSM 3677 sind.

5. Verwendung eines wäßrigen Nährmediums nach einem oder mehreren der Ansprüche 1 bis 4, dadurch gekennzeichnet, daß die untere Grenze der Keimdichte der homofermentativen Milchsäurebakterien bei Beginn der Züchtung zweckmäßig etwa 10⁴/ml und die obere Grenze etwa 10⁷/ml beträgt.

6. Verwendung einer Milchsäurebakterienkultur erhalten nach einem oder mehreren der Ansprüche 1 bis 5, als Silierhilfsmittel.

7. Trockennährmedium-Milchsäurebakterien-Kombinationspräparat, dadurch gekennzeichnet, daß es Alkalisalze der Essigsäure, Glucose, Pufferverbindungen, homofermentative Milchsäurebakterien in einer Menge von etwa 2,5 x 10⁴ bis 2,5 x 10⁷ pro 100 g Glucose sowie weitere Kohlenstoffquellen, Stickstoffquellen, Macroelemente und Spurenelemente in solchen Mengen enthält, daß nach Lösen des Präparats in Wasser das wäßrige Nährmedium nach Anspruch 1 erhalten wird.

## Claims

1. Use of an aqueous nutrient medium containing carbon sources, wherein monosaccharides are present in an amount of 2 to 6 wt.% based on the amount of water, nitrogen sources, macroelements and trace elements, alkali metal salts of acetic acid in an amount such that from 0.7 to 2.0 wt.% of CH₃COO groups is present, based on the amount of water, and buffer compounds in an amount such that, on standing for two days at 20°C with lactic acid bacteria added, the pH does not fall below 4, for the proliferation of homofermentative lactic acid bacteria under non-sterile conditions.

2. Use according to claim 1, characterized in that glucose is used as the monosaccharide.

3. Use of an aqueous nutrient medium according to one or both of claims 1 and 2, characterized in that the homofermentative lactic acid bacteria belong to homofermentative representatives of the genus Lactobacillus and/or Pediococcus.

4. Use of an aqueous nutrient medium according to one or more of claims 1 to 3, characterized in that the homofermentative lactic acid bacteria are Lactobacillus plantarum DSM 3676 and/or Lactobacillus plantarum DSM 3677.

5. Use of an aqueous nutrient medium according to one or more of claims 1 to 4, characterized in that the lower and upper limits of the microbial density of the homofermentative lactic acid bacteria at the beginning of cultivation are expediently about 10⁴/ml and about 10⁷/ml respectively.

6. Use of a lactic acid bacterial culture, obtained according to one or more of claims 1 to 5, as an ensilage aid.

7. Combination preparation of dry nutrient medium and lactic acid bacteria, characterized in that it contains alkali metal salts of acetic acid, glucose, buffer compounds and homofermentative lactic acid bacteria in an amount of about 2.5 x 10⁴ to 2.5 x 10⁷ per 100 g of glucose, and also contains other carbon sources, nitrogen sources, macroelements and trace elements in amounts such that the aqueous nutrient medium according to claim 1 is obtained after the preparation has been dissolved in water.

## Revendications

1. Utilisation d'un milieu nutritif aqueux contenant des sources de carbone, dans lequel des monosaccharides sont présents en une quantité de 2 à 6 % en poids par rapport à la quantité d'eau, des sources d'azote, des macroéléments et des oligoéléments, des sels de métaux alcalins de l'acide acétique en une quantité telle que l'on ait de 0,7 à 2,0 % en poids du groupe CH₃COO par rapport à la quantité d'eau, et des substances tampon en une quantité telle que, après deux jours d'abandon à 20°C, l'addition de ferments lactiques ne conduise pas à un pH inférieur à 4, pour assurer la prolifération de ferments lactiques homofermentatifs dans des conditions non-stériles.

2. Utilisation selon la revendication 1, caractérisée en ce qu'on utilise du glucose en tant que monosaccharide.

3. Utilisation d'un milieu nutritif aqueux selon une ou plusieurs des revendications 1 ou 2, caractérisée en ce que les ferments lactiques homofermentatifs appartiennent à des représentants homofermentatifs du genre Lactobacil-lus et/ou Pediococcus.

4. Utilisation d'un milieu nutritif aqueux selon l'une ou plusieurs des revendications 1 à 3, caractérisée en ce que les ferments lactiques homofermentatifs sont Lactobacillus plantarum DSM 3676 et/ou Lactobacillus plantarum DSM 3677.

5. Utilisation d'un milieu nutritif aqueux selon l'une ou plusieurs des revendications 1 à 4, caractérisée en ce que la limite inférieure de la densité de germes des ferments lactiques homofermentatifs est avantageuse-ment, au début de la culture, d'environ 10⁴/ml, et la limite supérieure est d'environ 10⁷/ml.

6. Utilisation d'une culture de ferments lactiques, obtenue selon l'une ou plusieurs des revendications 1 à 5, en tant qu'auxiliaire d'ensilage.

7. Préparation combinée d'un milieu nutritif sec et de ferments lactiques, caractérisée en ce qu'elle contient des sels de métaux alcalins de l'acide acétique, du glu-cose, des composés tampon, des ferments lactiques homo-fermentatifs en une quantité d'environ 2,5.10⁴ à 2,5.10⁷ par 100 g de glucose, ainsi que d'autres sources de car-bone, sources d'azote, macroéléments et oligoéléments, en des quantités telles que, après dissolution de la prépa-ration dans l'eau, on obtienne le milieu nutritif aqueux selon la revendication 1.
